# EUROPEAN PATENT SPECIFICATION

(11) **EP 2 996 577 B1**
(45) Date of publication and mention of the grant of the patent: **15.01.2020**
(21) Application number: 14791885.8
(22) Date of filing: 29.04.2014
(51) Int. Cl.: A61B 17/04, A61B 17/064, A61B 17/068, A61B 17/08, A61B 17/11, A61B 90/00, A61F 2/08

(54) **PERCUTANEOUS TENDON-MUSCLE-LIGAMENT APPROXIMATION DEVICE**
PERKUTANE SEHNEN-MUSKEL-LIGAMENT-ANNÄHERUNGSVORRICHTUNG
DISPOSITIF DE RAPPROCHEMENT PERCUTANÉ DE TENDON-MUSCLE-LIGAMENT

(30) Priority: 29.04.2013 US 201361817185 P
(43) Date of publication of application: 23.03.2016
(73) Proprietor: Regenexx, LLC, Des Moines, Iowa 50321 (US)
(72) Inventor: CENTENO, Christopher, Broomfield, Colorado 80021 (US); REISCHLING, Patrick, Broomfield, Colorado 80021 (US)
(74) Representative: Mewburn Ellis LLP
(86) International application number: PCT/US2014/035867
(87) International publication number: WO 2014/179310

(56) References cited:
- WO-A1-2009/046126
- WO-A1-2009/046126
- WO-A2-00/74579
- US-A1- 2009 287 304
- US-A1- 2009 287 304
- US-A1- 2010 049 320

## Description

### FIELD OF THE DISCLOSURE

The embodiments disclosed herein generally relate to devices and methods for treatment of damaged tendon, muscles, and ligaments, and more particularly, to devices and methods for the repair or stabilization of a torn, ruptured or otherwise damaged tendon, muscle, or ligament.

### BACKGROUND

Tendon, muscle, and ligament ruptures and tears occur in middle aged adults and younger athletes. For example, the annual incidence of Achilles tendon ruptures has been estimated to range from 5.5 to 9.9 ruptures per 100,000 people in North America. Rotator cuff tears also occur in patients in up to 20% of the population after age 32 years and in patients after age 60 having a tear up to 80% of the time. In addition, ligament tears are also common injuries. For example, one of the most common knee injuries is an anterior cruciate ligament sprain or tear. Athletes who participate in high demand sports like soccer, football, and basketball are more likely to injure their anterior cruciate ligaments. There are an estimated 80,000 to 100,000 anterior cruciate ligament (ACL) repairs performed in the United States each year.

Surgeries for these tendon, ligament, and muscle tears are often invasive. For example, with respect to a knee ACL, surgery can involve open or arthroscopic incisions that expose the ligament, typically followed by ligament replacement. ACL surgery often involves drilling a tunnel to hold the new ACL graft and can produce scar tissue that may reduce range of motion. Surgical replacement of the ACL has been associated with osteoarthritis over time and the ligament that is replaced often does not have the same bio mechanical properties as the original ACL.

The embodiments disclosed herein are directed toward overcoming one or more of the problems discussed above.

WO 00/74579 discloses instrumentation for cutting an opening in a side wall of a body conduit, such as an aorta, in a patient.

US 2010/049320 discloses apparatus and methods for repairing damaged tendons or ligaments. The apparatus includes an elongate tensile member and a pair of anchor assemblies connected for movement along the tensile member on either side of a repair site, such as a tear or laceration.

WO 2009/046126 discloses a device for endoscopy or endosonography-guided transluminal interventions whereby two luminal structures in the body may be drawn toward each other and a fluid conduit formed in between.

US 2009/287304 discloses a medical apparatus positionable in a cavity of a heart for constricting a mitral valve. The medical apparatus includes tissue anchors that are implanted in the annulus of the orifice. The tissue anchors are guided into position by an intravascularly or percutaneously deployed anchor guiding frame.

### SUMMARY OF THE DISCLOSED EMBODIMENTS

The disclosed embodiments include systems useful for the repair or stabilization of a torn, ruptured or otherwise damaged tendon, muscle, or ligament tissue. Repairs, stabilization, enhancement or other treatment can be made on the torn, ruptured or damaged tendon, muscle, or ligament of a human being or non-human animal. The described devices and methods may be implemented percutaneously.

The present invention provides a tissue approximation system as set out in claim 1.

Merely by way of example, a device in accordance with one set of embodiments comprises a hollow tube capable of percutaneous use, the hollow tube having a distal portion. The hollow tube houses an inner core which may be moved within the hollow tube between first and second positions. The inner core comprises a distal tip. When the inner core is placed into the first position, the distal tip extends away from or outside of the hollow tube such that the distal tip of the inner core may become securely attached to a selected tissue at a desired location. Once secured to tissue, the distal tip is configured to approximate the torn tissue in a selected direction when the device is pushed or pulled. The device also comprises at least one device control which may be used to place the inner core in the first or second position and thereby cause the distal tip to be deployed outside the hollow tube or to be retracted fully inside the hollow tube thereby releasing any attachment to the selected tissue. A stabilizer is provided in association with the hollow tube which in use abuts a surface to stabilize the hollow tube with respect to the tissue.

An alternative device embodiment includes a hollow tube and inner core which may be moved within the hollow tube between first and second positions. In the alternative embodiment however, one or more tines are formed in a wall of the hollow tube at a distal location. When the inner core is moved into the first position the tines are forced away from the hollow tube such that the tines may secure tissue. In this configuration, the device may be used to approximate the tissue secured by the tines. When the inner core is moved to a second position the tines retract to a position adjacent to the balance of the wall of the hollow tube. Therefore, when the inner core is moved to the second position the device may be withdrawn from the tissue. These alternative device embodiments also include a stabilizer and may also include device control as noted above.

With respect to either class of device embodiment, a substance, including but not limited to a therapeutic substance, a biological glue, a drug, a growth factor, stem cells or other substances may be injected into or placed upon the tissue through the hollow tube. In certain embodiments the hollow tube has multiple lumens with at least one lumen housing the inner core and another lumen providing for the injection or placement of a substance.

The tissue approximation system may comprise selected additional components including but not limited to a frame, additional one or more stabilizers, one or more delivery devices, and at least one imaging device.

Also disclosed herein are methods of using a tissue approximation device or system to secure selected biological tissues and approximate or otherwise move same. One representative method includes the steps of inserting the hollow tube of a tissue approximation device into a retracted tissue, deploying the distal tip of an inner core from the hollow tube to secure the tissue and pulling or pushing the tissue approximation device to move the retracted tissue as required to achieve therapeutic goals. The method may also include other steps including but not limited to applying a substance to the tissue, or retracting the distal tip of the inner core into the hollow tube thereby releasing the tissue and permitting withdrawal of the device.

Various modifications and additions can be made to the embodiments discussed without departing from the scope of the invention. For example, while the embodiments described above refer to particular features, the scope of this invention also includes embodiments having different combination of features and embodiments that do not include all of the above described features.

### BRIEF DESCRIPTION OF THE DRAWINGS

Fig. 1 is a schematic diagram of a percutaneous tendon-muscle-ligament approximation device having a hollow tube, inner core, and distal tip according to one embodiment;
Fig. 2 is a schematic diagram showing a percutaneous tendon-muscle-ligament approximation device with control means according to one embodiment;
Figs. 3A-3C are schematic diagrams showing a distal tip of a percutaneous tendon-muscle-ligament approximation device at various positions of a control dial, according to one embodiment;
Figs. 4A-4C are schematic diagrams showing an alternative device configurations fixed against the skin according to various embodiments;
Figs. 5A-5K are alternative distal tip configurations according to various embodiments;
Fig. 6 is a shoulder MRI demonstrating the placement and positioning of a percutaneous tendon-muscle-ligament approximation device according to one embodiment;
Fig. 7 is an ankle MRI demonstrating the placement and positioning of a percutaneous tendon-muscle-ligament approximation device according to one embodiment;
Fig. 8 is a schematic diagram according to an alternative embodiment of the device which includes a frame to hold the body part being treated;
Fig. 9 is a detailed view of a syringe locked in the frame with multi-port needle used to inject glue into a tendon tear;
Figs. 10A-10D are schematic diagrams of various hollow tube and distal tip assemblies of the percutaneous tendon-muscle-ligament approximation device according to various embodiments; and
Fig. 11A and 11B show various perspective views of a hollow tube of the percutaneous tendon-muscle-ligament approximation device, and a deployed hollow tube and distal tip assembly according to various embodiments.

### DETAILED DESCRIPTION

Unless otherwise indicated, all numbers expressing quantities of ingredients, dimensions reaction conditions and so forth used in the specification and claims are to be understood as being modified in all instances by the term "about".

In this application and the claims, the use of the singular includes the plural unless specifically stated otherwise. In addition, use of "or" means "and/or" unless stated otherwise. Moreover, the use of the term "including", as well as other forms, such as "includes" and "included", is not limiting. Also, terms such as "element" or "component" encompass both elements and components comprising one unit and elements and components that comprise more than one unit unless specifically stated otherwise.

Medicine has, in the past several decades, seen a rise in percutaneous procedures that have supplanted more invasive open surgical procedures. For example, open heart surgery has been largely replaced by the percutaneous insertion of cardiac stents. Orthopedic medicine has begun to follow this same trend. Recent advances in biologics have allowed surgeons to treat through injection, conditions once treated surgically, such as chronic epicondylitis.
The embodiments disclosed herein provide various alternative apparatus, devices and systems for percutaneous approximation and fixation of a complete, retracted tendon, ligament, or muscle tear. The disclosed embodiments facilitate procedures which can be performed under imaging guidance without the need for open surgery.

### Percutaneous Tendon-Muscle-Ligament Approximation Device

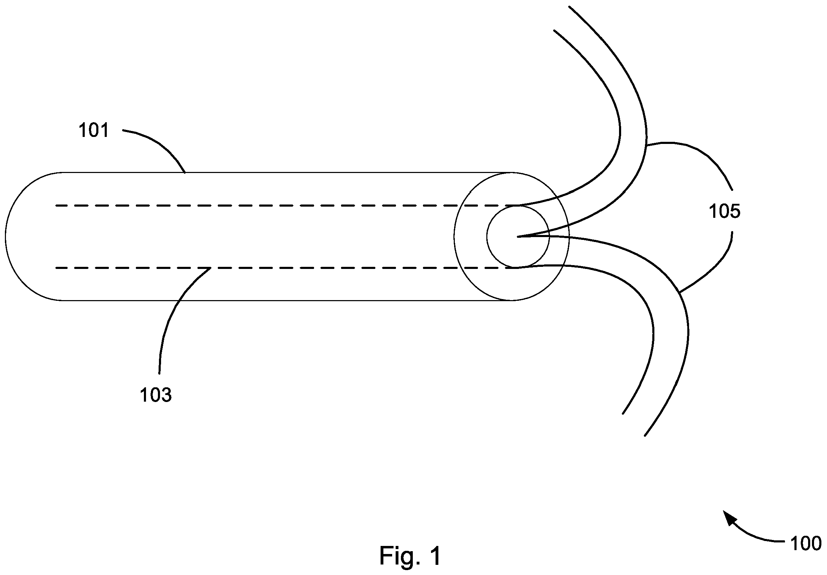

Fig. 1 is a schematic perspective view of the distal end of a representative, cannula and catheter assembly 100 of a tendon-muscle-ligament (TML) approximation device according to various embodiments. The distal end 100 includes a hollow tube 101. The hollow tube 101 houses and/or guides an inner core 103. The hollow tube 101 can be any type of hollow structure able to house an inner core 103. This includes, for example, an introducer needle, a cannula, a lumen, sheath, etc. However, it is to be understood that these examples are not limiting examples, and that other structures may be used.

The distal portion of the hollow tube 101 can be sharp or blunt. In some embodiments, the inner core 103 is sharpened on its distal tip and this slightly extends beyond the distal tip of the hollow tube 101. This allows the distal end 100 of the approximation device to pierce tissue. In other embodiments, a stylet is used for the same purpose and the inner core 103 is later inserted inside the hollow tube 101. In other embodiments, the distal tip of the hollow tube is blunt and in order to pierce the skin, a scalpel is used to open a small incision.

In some embodiments, the hollow tube 101 of the TML approximation device may have multiple lumens that are configured to deliver medications or biologics through the TML approximation device or to visualize the site using a small diameter arthroscope. Direct visualization of the torn site through fiber optics or other means would allow tendons, ligaments, or muscles enclosed in bone to be visualized, as ultrasound would not be useful in treating this type of tear. In particular, this would be very helpful in approximating a torn ACL ligament, where ultrasound couldn't be used to visualize the tear because the ACL is enclosed in the trochlear groove. While x-ray may be helpful in localizing the TML approximation device in the knee, it wouldn't be helpful in visualizing the tear and placing the tendon approximation device securely into the torn ligament ends, as the ligament would not visible using radiography. However, direct visualization would allow this to occur. In other embodiments, the TML approximation device is designed so that it can be used in conjunction with a separate small diameter arthroscopy system.

The hollow tube 101 of the approximation device in some embodiments may be stainless or carbon steel, while in other embodiments more durable or harder metals may be needed. These may include, but are not limited to NiTinol, titanium alloys, or other alloys.

Distal tip 105 is located at the distal end 100 of the TML approximation device. The distal tip 105 includes hooks, barbs, or other anchoring means used to anchor the distal end 100 of the TML approximation device to tendon, muscle, ligament, or other tissue. As illustrated, distal tip 105 has two distal hooks deployed from the open end of cannula 101. In other embodiments, distal tip 105 may include hooks or barbs that do not deploy from the open end of cannula 101, and instead, or additionally, deploy from other openings in the walls of cannula 101, or protrude directly from the walls of cannula 101 itself. For example, in some embodiments, the hollow tube 101 is closed at its distal end (i.e. does not have an open end) and the distal tip 105 extrudes out of the side walls of the hollow tube 101. In such embodiments, the distal tip of the hollow tube 101 can be sharp, allowing it to pierce tissue. Furthermore, distal tip 105 deployment configurations may also be changed to suit the medical need.

The inner core 103 and/or distal tips 105 may be constructed of NiTinol or another memory metal or a metal or other material that would allow deployment from the hollow tube. The type of metal or other material would also be selected to ensure the degree of stiffness required to anchor tissue as the TML approximation device is pulled while still allowing for retraction of a distal tip 105 within the hollow tube so that the TML approximation device can be removed easily from the tissue.

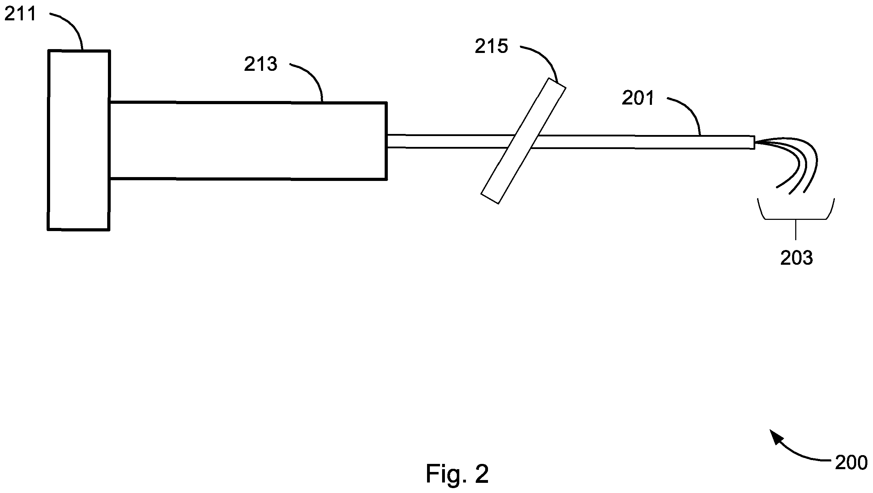

Fig. 2 is a schematic diagram of a TML approximation device 200 which includes a distal end, handle 213, device controls 211, and stabilizer 215. The distal end includes hollow tube 201 and distal tip 203. Distal tip 203 has a rake deployment configuration with three tines. The handle 213 provides an area for a physician to grip while handling the TML approximation device. The handle 213 is used by the physician to pull or push the tissue or direct the hollow tube. In various embodiments, the handle 213 also houses mechanisms for advancing and retracting an inner core within the hollow tube 201, and for deploying distal tip 203. However, some embodiments don't employ a handle 213 and instead the physician manipulates the proximal end of the inner core through hollow tube 201 by pushing or pulling it to deploy or retract the distal tip 203. In some embodiments the proximal end of the inner core has a tab which can be manipulated by the physician.

Device controls 211 allow the control of inner core advancement and retraction, and/or distal tip deployment and retraction. Device controls 211 are configured to lock the distal tip 203 in both a deployed position and a retracted position. Device controls 211 may include, but are not limited to, a locking dial, continuous dial, flip switches, toggles, and buttons. Device controls 211 include mechanical, electronic, and electromechanical control means. In various embodiments, device controls 211 can be located at either the proximal end, or in alternative embodiments, in the mid-section of the TML approximation device, such as the handle 213.

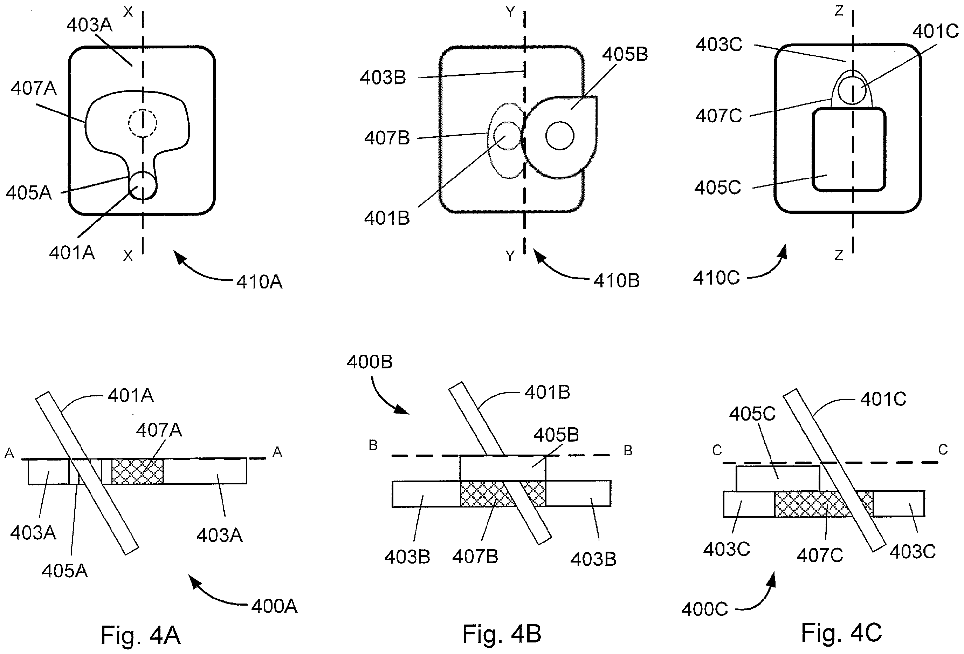

Stabilizer 215 allows the TML approximation device to stay fixed against the skin of a patient and steadies the TML approximation device to prevent it from moving once placed in a desired position by the physician. In some embodiments, the stabilizer 215 also stabilizes the position of the hollow tube 201 in relation to the entry site in the skin of the patient. Further configurations and embodiments of the stabilizer 215 are described in more detail below with respect to Figs. 4A-4C.

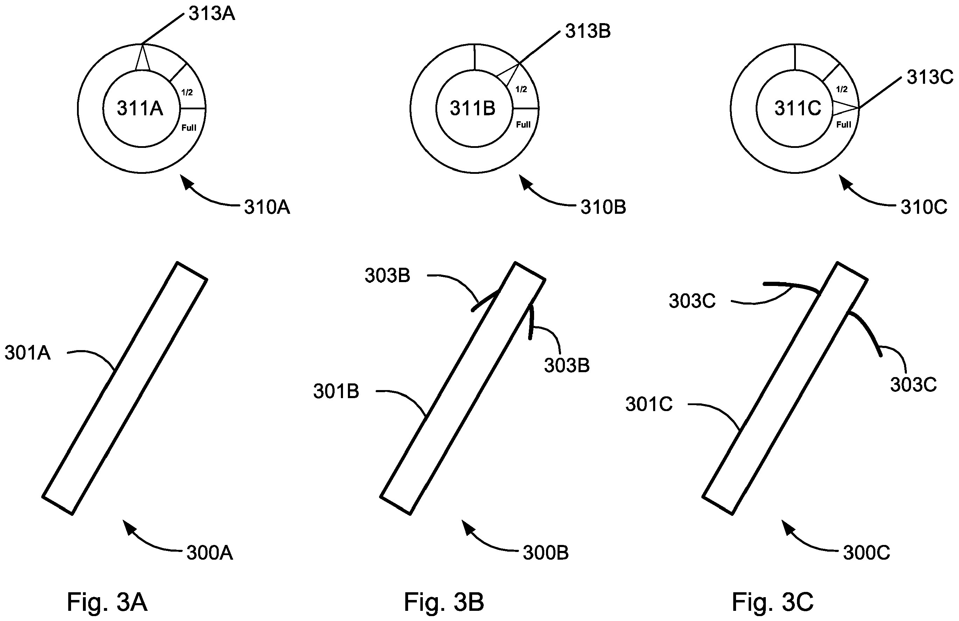

Fig. 3 illustrates device controls in the form of a dial at various positions 310A-310C and a corresponding configuration of a distal end of the TML approximation device 300A-300C. For example, Fig. 3A illustrates device controls 310A in the form of dial 311A. Dial 311A is turned to the retracted position setting 313A. Corresponding to the retracted position setting 313A, distal end 300A shows a hollow tube 301A having no distal tip deployment. Next, Fig. 3B illustrates device controls 310B having dial 311B. Dial 311B is turned to a "1/2" deployment setting 313B. Corresponding to the "1/2" deployment setting 313B, distal end 300B illustrates hollow tube 301B having distal tip 303B with two half (i.e. partially) deployed hooks from the walls of hollow tube 301B. Finally, Fig. 3C depicts device controls 310C having dial 311C turned to the "full" or fully deployed setting 313C. Corresponding to the "full" deployment setting 313C, distal end 300C depicts a hollow tube 301C having distal tip 303C with two fully deployed hooks from the walls of hollow tube 301C.

Certain medical applications may also require more or less deployment of the distal tip 310A-310C. For example, the location of nerves or blood vessels seen on ultrasound in direct proximity to the distal tip 310A-310C may necessitate that the tip have a variable deployment length. In other embodiments, for example, the tip may be deployed in other increments, including, but not limited to, "1/4," or "3/4" of the full length of the distal tip, or be deployed only from the side of the TML approximation device that is not in close proximity to such critical structures.

Figs. 4A-4C illustrates alternative embodiments of the stabilizer 215 with reference to Fig. 2, from a side view and top view. Fig. 4A illustrates a cross-sectional side view 400A of stabilizer 403A, taken along line X-X, which can be seen in cross-sectional top view 410A. In side view 400A, hollow tube 401A is snapped into place in a smaller diameter locking position 405A. The back wall of a working position 407A is visible, before again encountering stabilizer body 403A. Top view 410A shows a cross section taken along line A-A, visible in side view 400A. In top view 410A, stabilizer 403A is shown having larger diameter working position 407A and narrower diameter locking position 405A. Hollow tube 401A is clearly in locked position 405A having a smaller diameter than working position 407A. The positioning of the hollow tube 401A in working position 407A is depicted in phantom lines.

Fig. 4B illustrates a cross-sectional side view 400B of stabilizer 403B, taken along line Y-Y, which can be seen in cross-sectional top view 410B. In side view 400B, hollow tube 401B is shown positioned within working area 407B. A cam lock 405B is used to lock hollow tube 401B into place. Cam lock 405B can rotate to apply pressure against the hollow tube 401B to lock it into place against the walls of working area 407B. Top view 410B shows a cross section taken along line B-B, visible in side view 400B. In top view 410B, stabilizer 403B is shown with hollow tube 401B located within working area 407B. Cam lock 405B is shown attached to one side of the stabilizer 403B from which the cam lock 405B can rotate to lock hollow tube 401B into place.

Fig. 4C illustrates a cross-sectional side view 400C of stabilizer 403C, taken along line Z-Z, which can be seen in cross-sectional top view 410C. In side view 400C, hollow tube 401C is shown positioned within working area 407C, and leaning against the sliding lock 405C. The sliding lock 405C can be pushed against hollow tube 401C (i.e. in the left-right direction relative to the side-view 400C perspective) to hold the hollow tube 401C in place against the wall of working area 407C. Top view 410C shows a cross section taken along line C-C, visible in side view 400C. In this view, the sliding lock is pushed in the up-down direction to lock the hollow tube 401C in working area 407C.

Each of stabilizers 403A-403C are capable of locking hollow tubes 401A-401C, respectively, against the skin to fix the position of the tissue for treatment. In the depicted side view embodiments 400A-400C, the stabilizers would be positioned against the skin such that the skin would be positioned opposite the stabilizers from cross-sectional lines A-A, B-B, and C-C respectively.

In some embodiments, the stabilizers may also serve a stereotactic function, where certain coordinate measurements are placed on the apparatus which allow the TML approximation device to be guided to a certain location in a tendon, muscle, or ligament via a digitized three dimensional MRI or other imaging study. Furthermore, in some embodiments, the stabilizer itself is affixed to the skin with adhesive or sutures or some other type of anchoring system.

It is to be understood that the above embodiments are described by way of illustration only, and that other means of stabilizing the hollow tube may be used. For example, in certain other embodiments, a screw may be used as a stabilizer that locks the approximation device to the skin anchor.

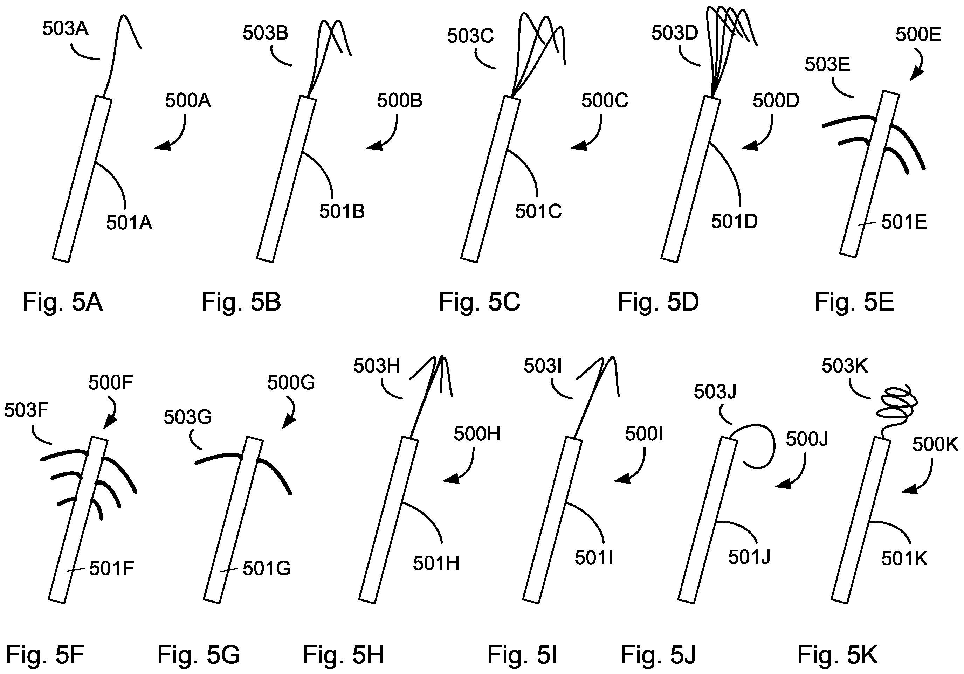

Figs. 5A-5K depict various different distal tip deployment configurations. For example, Fig. 5A depicts a single hook deployment configuration 500A. Distal tip 503A has a single hook deployed from the open end of hollow tube 501A. Fig. 5B depicts a two-tine rake deployment configuration 500B. Distal tip 503B has two hooks deployed from the open end of hollow tube 501B in a rake-like arrangement. Fig. 5C depicts a three-tine rake deployment configuration 500C. Distal tip 503C has three hooks deployed from the open end of hollow tube 501C in a rake-like arrangement. Fig. 5D depicts a four-tine rake deployment configuration 500D. Distal tip 503D has four hooks deployed from the open end of hollow tube 501D in a rake-like arrangement. Fig. 5E depicts a four anchor barb deployment configuration 500E. Distal tip 503E has four barbs protruding from the sides of hollow tube 501E, two barbs on each of the opposite sides of hollow tube 501E. Fig. 5F depicts a six anchor barb deployment configuration 500F. Distal tip 503F has six barbs protruding from the sides of hollow tube 501F, three barbs on each of the opposite sides of hollow tube 501F. Fig. 5G depicts a two anchor barb deployment configuration 500G. Distal tip 503G has two barbs protruding from the sides of hollow tube 501F, one barb on each of the opposite sides of hollow tube 501G. In each of the anchoring barb deployment configurations 500E-500G, the hollow tubes 501E-501G may or may not have a distal open end. That is, the ends of hollow tubes 501E-501G may be closed off as the anchoring barbs deploy from the sides of hollow tubes 501E-501G, and not through a distal open end. Furthermore, in some embodiments, the anchoring barbs may themselves be smooth, the distal tip itself acting as a barb, or in alternative embodiments, the anchoring barbs may themselves have a barb-like structure. Fig. 5H depicts a grappling hook deployment configuration 500H. Distal tip 503H has three hooks on its end, and is deployed from the open end of hollow tube 501H. The three hooks of distal tip 503H deploy from the open end in a grappling-hook configuration, i.e. spaced having roughly an equal radial distance from one another. The three hooks may be three separate hooks, as in three-tine rake deployment configuration 500C, or may have a single common body with three hooks at the end. Fig. 5I depicts an alternative grappling hook deployment configuration 5001. Distal tip 5031 has two hooks on its end, and is deployed from the open end of hollow tube 5011. The two hooks of distal tip 5031 deploy from the open end in a grappling-hook configuration, i.e. facing opposite each other. The two hooks may be two separate hooks, as in two-tine rake deployment configuration 500B, or may have a single common body with two hooks at the end. Fig. 5J depicts a loop deployment configuration 500J. Distal tip 503J has a loop shape that deploys from the open end of hollow tube 501J. In other embodiments, distal tip 503J can deploy from the sides of hollow tube 501J. In some embodiments, distal tip 503J has a shape such that, when extended fully, it will loop back onto itself. In certain embodiments, the distal tip 501J can loop back onto itself such that distal tip 503J can insert itself back into a locking mechanism in the side of hollow tube 501J, thereby locking the loop into place. Fig. 5K depicts a corkscrew deployment configuration 500K. Distal tip 503K deploys in a corkscrew arrangement that deploys from the open end of hollow tube 501K. The cork screw shape of distal tip 503K bores into tissue, securing the distal end of the TML device to the tissue.

The above descriptions are not meant to be an exhaustive list of distal tip deployment configurations, and other distal tip deployment configurations may be used that are capable of attaching, grabbing, or otherwise securing the distal end of the TML approximation device to tissue. For example, in alternative embodiments, a small pincer may be deployed to pinch tissue. In other embodiments, the distal tip deployment configurations may be altered to be more effective in a pushing direction as opposed to a pulling direction. In yet further embodiments, the distal tip deployment configurations may include a distal and proximal hooks configured to attach to both sides of torn tissue.

### Systems and Treatments using TML Approximation Device

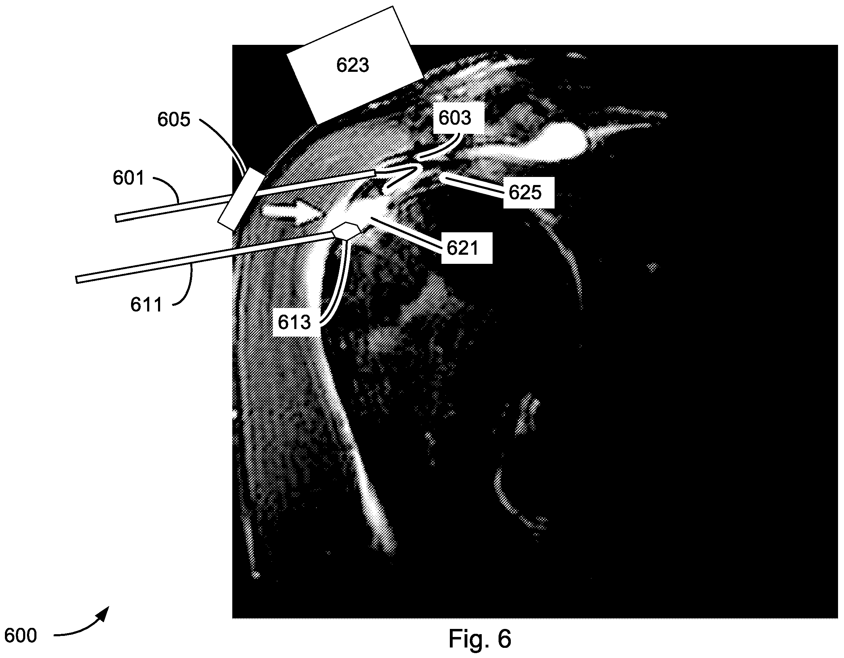

Fig. 6 depicts a shoulder MRI demonstrating the use of a TML approximation device for treatment of a torn supraspinatus tendon 600. Hollow tube 601 is illustrated inserted into the patient. The hollow tube 601 is attached to the upper half of torn tendon 625 via distal tip 603. Distal tip 603 has a single hook deployment configuration. The hollow tube 601 is further fixed against the skin of the patient and can be locked into place via stabilizer 605. A second needle 611 is inserted into the treatment site 621 to deliver medication or glue 613, exiting at the tip of needle 611. An ultrasound imaging transducer 623 is positioned to observe the hollow tube 601 being placed into the torn and retracted tendon 625.

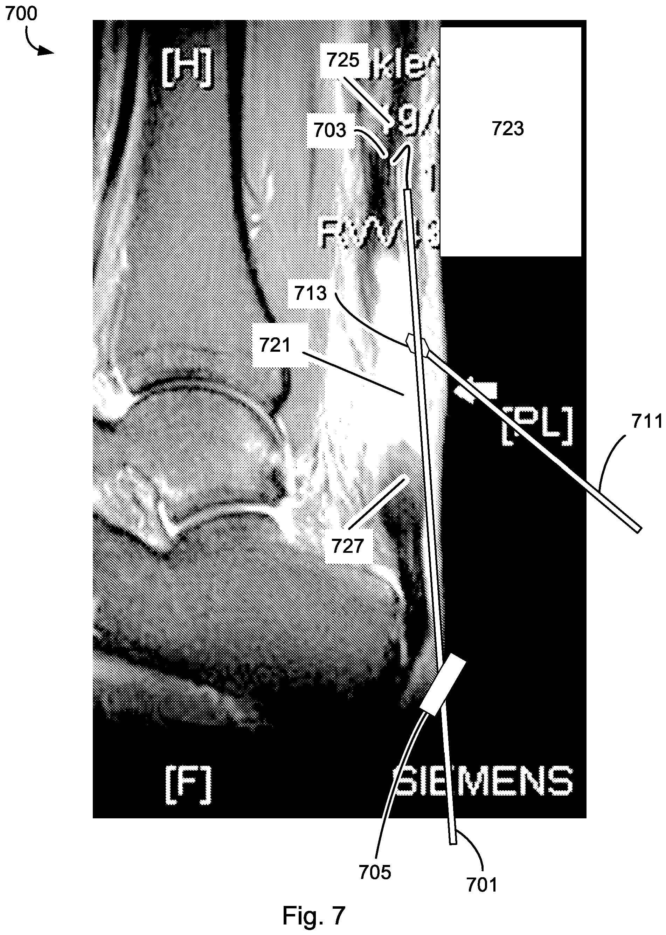

Fig. 7 depicts an ankle MRI demonstrating the use of a TML approximation device from the treatment of a torn Achilles tendon 700. Hollow tube 601 is illustrated inserted into the patient. The hollow tube 701 is attached to the upper retracted tendon 725 via the distal tip 703. Distal tip 703 has a single hook deployment configuration. The hollow tube 701 is further fixed against the skin of the patient and can be locked into place via stabilizer 705. The hollow tube can be pulled inferior to drag the tendon back into place against lower retracted tendon 727. A second treatment needle 711 is inserted into treatment site 721 to deliver medication or glue 713 that exits from the tip of needle 711. An ultrasound imaging transducer 723 is positioned to allow direct visualization of the TML approximation device placement and tendon re-approximation.

In one aspect disclosed herein, the TML approximation device is guided under ultrasound imaging into the retracted tendon 625, 725 and then the distal tip 603, 703 is deployed to anchor the distal end of the TML approximation device into the tendon. The hollow tube 601, 701 is then pulled and the retracted tendon 625, 725 is approximated to its other side 727. Once the retracted ends are approximated, a locking device 605, 705 on the skin is used that keeps the hollow tube 601, 701 of the TML approximation device fixed so that the ends of the tendon do not retract back into the body. Ultrasound imaging is then again used to visualize the tear area and a second needle 611, 711 or other percutaneous delivery device can be inserted to inject biologic glue 613, 713. Another adherence system could also be used instead of glue to percutaneously staple, suture, or otherwise fix the tendon pieces together. All of this is accomplished without the need for open surgery.

In some embodiments, two TML approximation devices may be required, with one TML approximation device on each side of the retracted tendon 725 and 727. These two devices would pull or push the tendon ends in the opposite direction toward approximation. In one embodiment, an introducer needle of the tendon approximation device can also deliver the biologic glue or another substance through holes in the distal hollow tube portion of the TML approximation device so that there is no need for a second needle to inject this substance. In another embodiment, there is a specialized percutaneous introducer that can work alone, with a skin anchor, or with a frame. This needle has multiple holes in its distal end that allow glue or another substance to fill the gap in the tendon. One or more needles or a catheter can be used to thoroughly fill the tendon gap.

In other embodiments, the approximation device includes a mechanism whereby both ends of the retracted tendon 725, 727 are grabbed via distal and proximal hooks or other anchoring barbs and approximated mechanically while the inserted device remains stationary. This allows the physician to approximate tendons, ligaments, or muscles where both ends are retracted without having to use two tendon approximating devices.

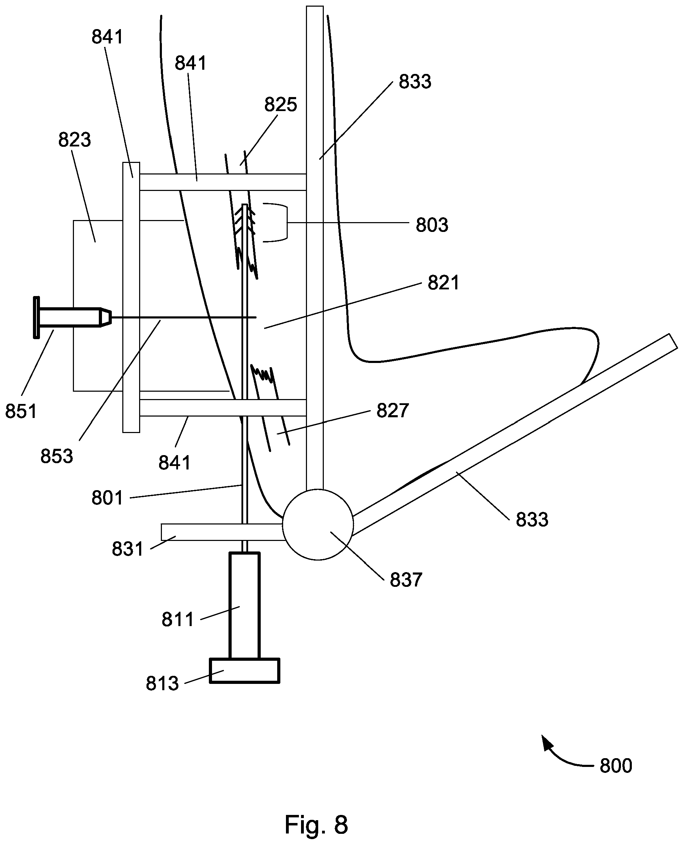

Fig. 8 schematically depicts the treatment of a torn Achilles tendon using the TML approximation device in conjunction with a frame 800. The TML approximation device includes a distal end, handle 811, and device controls 813. The distal end includes hollow tube 801 and distal tip 803. Distal tip 803 has six anchor barb deployment configuration with three barbs on either side of the hollow tube 801. Distal tip 803 attaches hollow tube 801 of the distal end of the TML approximation device to upper torn tendon 825. A frame is provided to hold the body part being treated. The frame includes a hinge 837 that allows the foot platform to be moved so that it can either dorsi-flex or plantar-flex the ankle. The sole of the foot rests upon the foot platform 833. The frame also comprises a stabilizer 831 that holds the TML approximation device in place such that the tendon cannot be retracted once pulled into position. The frame also includes a vertical support 833 to provide support to the ankle frame, as well to provide lateral stability to the foot and ankles. The frame also includes an ankle frame 841 that surrounds the foot and ankle. In some embodiments, the ankle frame 841 is configured to have a separate stabilizer to support syringe 851, and/or ultrasound transducer 823. Syringe 851 comprises an introducer needle 853 which can be used to deliver either medication or biologic glue to treatment site 821.

In one embodiment disclosed herein, the tendon approximation device is held in place via stabilizer 831 of the frame. The frame helps position the extremity (such as a foot/ankle, knee or shoulder). The frame helps hold the tendon approximation device so that the tendon 825 doesn't retract back into the body. The frame may also allow the ultrasound transducer 823 or other imaging probe or device to be held stationary at the appropriate angle to facilitate visualization of the tendons 825, 827 and distal end of the TML approximation device. The frame also allows the syringe 851 to be held in place so that the biologic glue or other substance can be injected into the precise treatment site 821 of the tear (e.g. through ankle frame 841).

Once the approximation device is deployed in tissue, the tissue is then approximated by pulling or pushing on the TML approximation device. In some embodiments the torn tissue ends 825, 827 are pulled together, in other embodiments the tissue ends are pushed together. For example, it may be more helpful for the physician in some circumstances to push the tissue ends into direct approximation. This may be helpful where the density and nature of the tissue may withstand a gentle pushing force more than a pulling force.

The TML approximation device may be guided via ultrasound imaging and thus in some embodiments has a highly echogenic hollow tube portion 801 and/or distal tip section 803 to allow proper visualization. In addition, the angle of the deployment of the hollow tube 801 and distal tip 803 must be as parallel to the skin and ultrasound transducer as feasible so that they reflect ultrasound energy back to the transducer 823. Steeper angles that are closer to perpendicular to the skin surface are avoided as they will deflect ultrasound energy away from the transducer 823, reducing the visualization of the hollow tube 801 and distal tip 803. This impacts how a frame is utilized with the TML approximation device, as it will control the angle of insertion of the TML approximation device through the frame. Likewise, the exiting of the distal tip 803 from the hollow tube 801 will need to be in planes and at angles which maximize ultrasound energy return back to the transducer 823. In other embodiments, the hollow tube 801 or distal tip 803 are made of or coated with materials that are highly echogenic.

In other embodiments where the TML approximation device is guided under c-arm fluoroscopy or x-ray, the TML approximation device is constructed of materials which are radio-opaque. For example, the TML approximation device may have certain radio-opaque markers on the distal tip 803 that allow the physician to better visualize the distal tip 803. In one embodiment, the TML approximation device has graduated markers on the distal tip 803 which allows the physician to easily see how much the distal tip 803 is deployed into tissue. In yet other embodiments, the TML approximation device with hollow tube 801 and distal tip 803 may be imaged with other devices such as computed tomography or even via a small arthroscope I might.

In many embodiments, the TML approximation device will need to be inserted at angles that approximate the longitudinal axis of the tendon, ligament, or muscle. This should generally not exceed 45 degrees to allow the tissue to be approximated with the most efficiency.

Clinical uses for the TML approximation device include, but are not limited to treatment of full or partial thickness tendon tears, ligaments tears, or muscle tears. For example, a partial list of common ailments treated would include a full thickness retracted Achilles tendon tear, a similar rotator cuff tear, or a knee ACL or collateral ligament tear, a retracted hamstrings muscle, or biceps tendon or tendon/muscle tear.

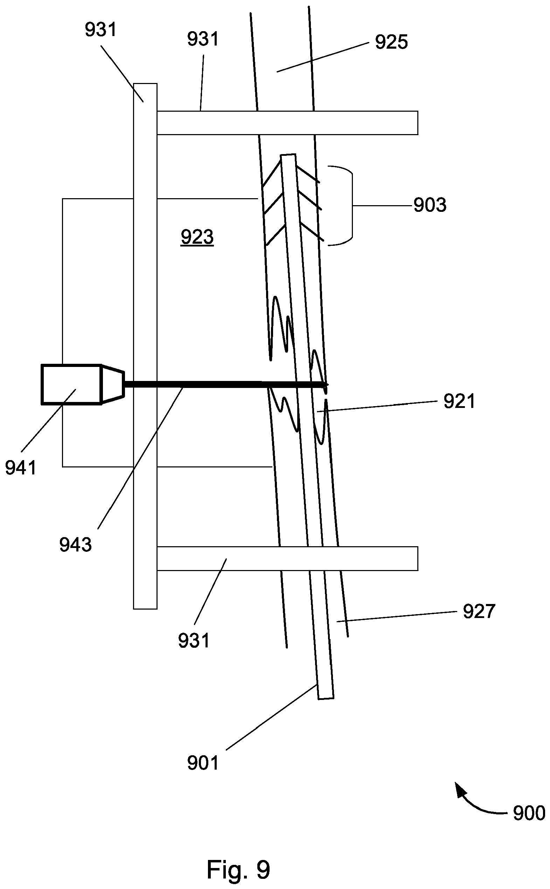

Fig. 9 depicts a detailed view of the Achilles tendon from Fig. 8 after being approximated 900. Upper torn tendon 925 and lower torn 927 are approximated together by the TML approximation device. The distal end of the TML approximation device is connected to upper torn tendon 925 via distal tip 903 deployed from hollow tube 901. Syringe 941 is locked in place by ankle frame 931. Syringe 941 has introducer needle 943 inserted into treatment site 921. The introducer needle is configured to deliver medication, or other biologics, such as biologic glue, to the treatment site 921. In some embodiments, the introducer needle 943 can be a multi-port needle with multiple ports along the walls of introducer needle 943. In other embodiments, the introducer needle 943 can be a traditional hypodermic needle. In yet other embodiments, the introducer needle 943 can comprise the same or similar structure as the hollow tube 901. In some alternative embodiments, the hollow tube 901 can be used to deliver the biologics to the treatment site 921 in place of separate syringe 941.

The tissue, once approximated, can be treated with a variety of items including fibrin or other biologic glues. Platelet rich plasma or stem cells may be added to the approximated tear with or without a scaffold material to enhance healing. For a tendon tear with a bone avulsion component a biologic or chemical bone cement may be added to the fibrin glue to enhance osteointegration. In addition, other growth factors or cytokines may be used to enhance healing such as, but not limited to bone morphogenic proteins (BMPs), fibroblast growth factor (FGF), transforming growth factor (TGF), vascular endothelial growth factor (VEGF), etc.

### Hollow Tube and Distal Tip Assemblies

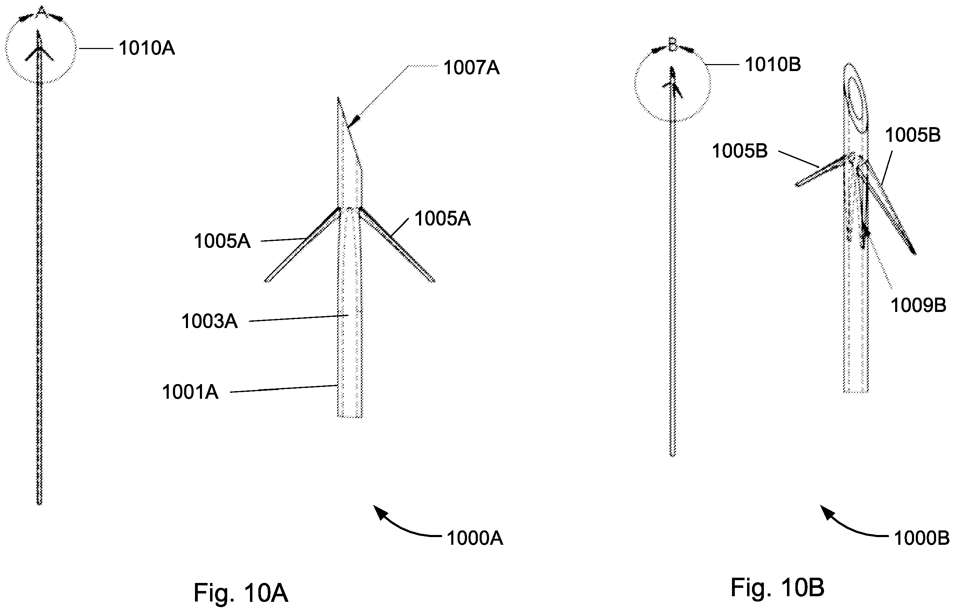

Figs. 10A-10D illustrate various hollow tube and distal tip assemblies, according to various embodiments. Fig. 10A illustrates side view of a hollow tube and distal tip assembly 1000A. In particular, distal end 1010A is shown in a close-up view. Distal end 1010A comprises hollow tube 1001A, inner core 1003A, distal tip comprising two tines 1005A deployed from either side of hollow tube 1001A. Hollow tube 1001A further includes a sharp tip 1007A.

In one embodiment, the hollow tube 1001A has a total length 6 inches (15.2 cm), with an outer diameter (0OD) of 0.05 inches (0.127 cm), and an inner diameter (0ID) of 0.03 inches (0.07 cm). The sharp tip 1007A is configured to have an 18-degree bevel, with a length of 0.25 inches (0.63 cm) measured from the top of tines 1005A to the end of sharp tip 1007A. In other embodiments, the tip may include, but are not limited to Quincke, Sprotte, Touhy, Whitacre, or pencil point tips. The tines 1005A have a length of 0.22 inches (0.55 cm) and extend from the body of hollow tube 1001A at a 45-degree angle with respect to the longitudinal axis of the hollow tube 1001A.

Fig. 10B illustrates a schematic view of the hollow tube and distal tip assembly 1000B. In particular, distal end 1010B is shown in a close-up view with respect to tines 1005B and cut outs 1009B of hollow tube body. In one embodiment, V-shapes are cut out of the hollow tube body and tines 1005B are shape set, leaving cutouts 1009B in the hollow tube body. In some embodiments, the tines 1005B can be set out with a 45-degree angle with respect to the longitudinal axis of the hollow tube. In some embodiments, the V-shape cutout 1009B is laser cut into nitinol hollow tubing.

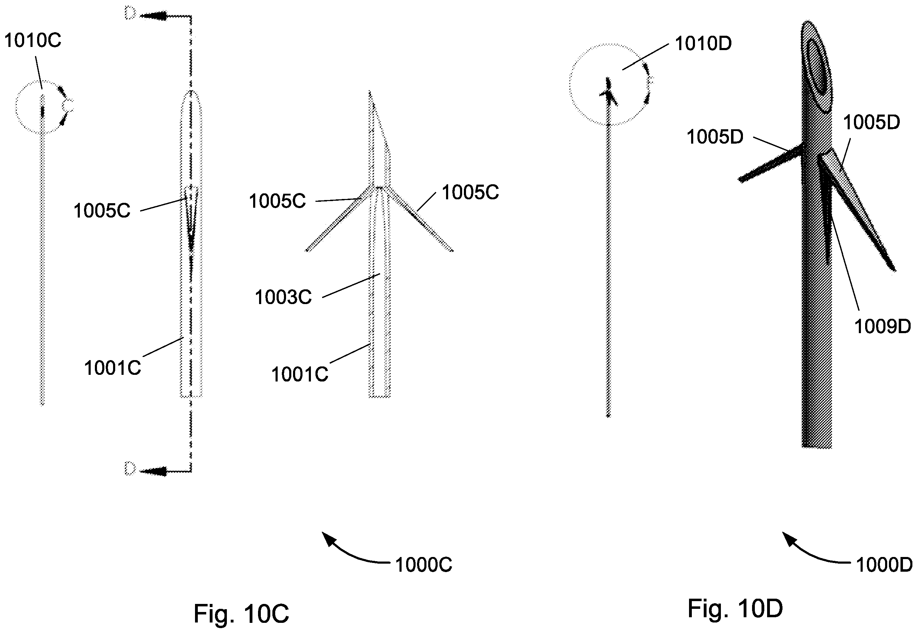

Fig. 10C illustrates a cross-sectional side view of the hollow tube and distal tip assembly 1000C. Distal end 1010C comprises a hollow tube 1001C having tines 1005C. A cross section is taken along line D-D. The cross-section illustrates a cross section of hollow tube 1001C, inner core 1003C and tine 1005C.

Fig. 10D illustrates a perspective view of the hollow tube and distal tip assembly 1000D. A close-up perspective view of distal end 1010D is presented. The close-up perspective view comprises cutouts 1009D and tines 1005D.

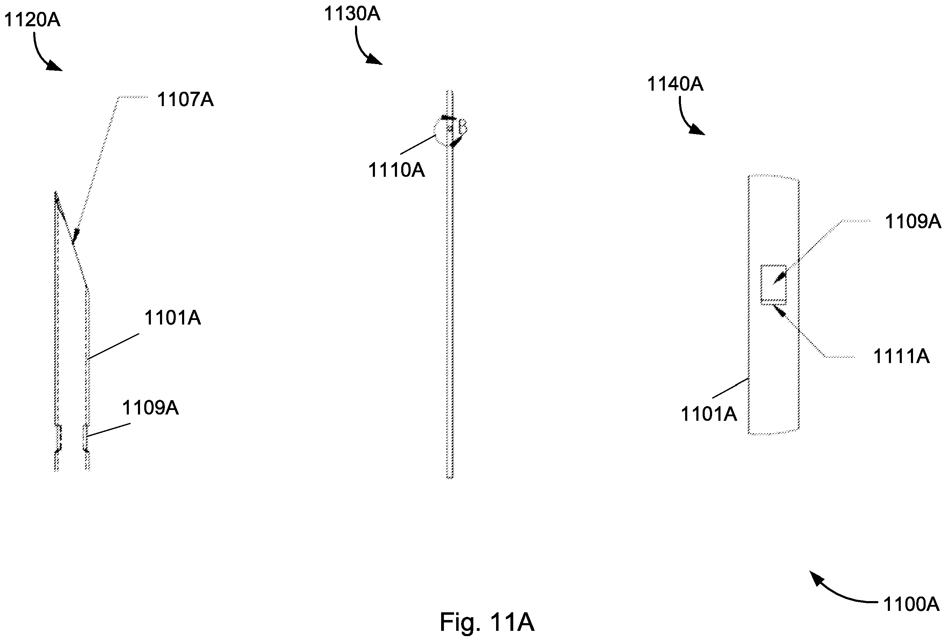

Fig. 11A illustrates an alternative embodiment of a hollow tube 1100A. The hollow tube 1100A has body/walls 1101A, with through port 1109A. Hollow tube 1100A also has sharp tip 1107A. A zoomed out view 1130A depicts the hollow tube having distal end 1110A. 1140A depicts a close-up view 1140A of distal end 1110A. The close-up view 1140A depicts hollow tube body/walls 1101A having through ports 1109A going completely through walls 1101A on both side of the hollow tube. Through ports 1109A also have angled chamfers 1111A.

In one embodiment, the total length of the hollow tube body 1101 A is 5 inches (12.7 cm) with an outer diameter (0OD) in the range of 0.0715 to .0724 inches (0.18161 to 0.18389 cm), and an inner diameter (0ID) in the range of 0.0595 to 0615 inches (12.7 cm) inches. The sharp tip 1107 A is configured to have an 18-degree bevel, with a length of 0.50 inches (1.27 cm) measured from the top of the through ports 1109 A to the end of sharp tip 1107 A. The through ports 1109A have a width of 0.035 inches (12.7 cm) and a length of 0.05 inches (12.7 cm) measured from the top of chamber 1111 A. The chamber 1111 A slopes downwards at a 45-degree angle relative to a longitudinal axis of the hollow tube body / walls 1101 A.

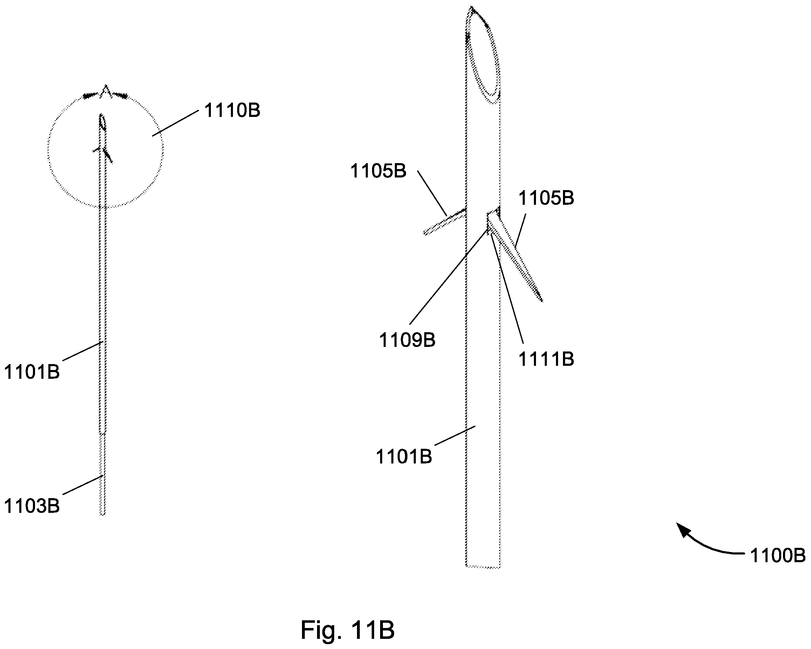

Fig. 11B illustrates a perspective view of a deployed hollow tube and distal tip assembly 1100B. The deployed assembly comprises hollow tube body 1101B, inner core 1103B, and distal end 1110B. Distal end 110B comprises hollow tube body 1101B, having through ports 1109B, the through ports having chamfers 1111B. Tines 1105B are deployed through the through ports 1109B.

### Example: Cadaver Testing of Tissue Approximation Device

Testing was conducted on an artificial tear in the patellar tendon of a cadaver knee to evaluate the performance of a TML approximation device as described herein.

The materials used in conducting the evaluation comprised a cadaver knee; a TML approximation device substantially as illustrated in Fig. 11 comprising: an outer cannula (i.e. hollow tube) weighing 16 grams, having a beveled tip, with two open through-ports at the distal end, and an inner needle, weighing 18g, constructed from nitinol, and having two tines to be deployed from the two through ports of the cannula; an ultrasound device, and a scalpel.

Three luer lock syringe caps were arranged on the outer tube of the tissue approximation device. One cap assembled to the proximal end of the outer cannula, a second cap to stop the tines from exceeding the distal tip of the cannula and a third cap at the proximal end of the inner needle to easily control deployment and attach a syringe if needed. The caps were glued at these locations using medical grade epoxy with a 24 hour incubation time at room temperature.

An incision to access the patellar tendon was made on the anterior side of the knee, medial to lateral, just below the patella. The patellar tendon was subsequently transected. The TML approximation device was visualized using ultrasound, which would be a standard imaging modality of the procedure.

The planned procedure was as follows: under ultrasound guidance the TML approximation device in the un-deployed state is to be introduced into the tissue from both the proximal and distal end of the tendon in subsequent tests. The TML approximation device will be maneuvered through both ends of the tendon traversing the tear. Once in position, the nitinol needle will be retracted from the cannula deploying the tines into the tissue. With the tines deployed, the clinician can approximate the ends of the tear together. Fibrin glue is to be administered to hold the approximation in place. Once the tissue is firmly glued the tines are pushed back into the cannula and the TML approximation device will be removed.

According to the above plan, the cadaver patellar tendon was transected and visualized under ultrasound. The needle was echogenic enough to be seen as it moved through the tissue. It appeared to the clinician that the selected TML approximation device final outer diameter, 16g, was too large for this particular location and tendon. This size needle would be appropriate for the Achilles tendon, but the patellar tendon is much smaller. Additionally the tissue of the cadaver was of poor quality as the specimen was frozen and thawed a couple days prior. It was also noted that the patient may have been bed ridden for some time prior to death based on the condition of the tendon.

The TML approximation device was able to penetrate the tendon although there were some initial problems with deployment. The epoxy of the luer lock caps on the TML approximation device did not hold very well and as a consequence made it difficult to deploy and un-deploy the tines. One device failed as the luer look caps detached from the needles. The second device stayed intact until the testing was completed. Initially it appeared that the deployed tines did not result in grasping the tissue. This may have been the result of the tines not deploying. With the second device, deployment was verified by ultrasound as the tines were visible under ultrasound and did result in significant snagging of the tissue. The tissue was able to be manipulated and pulled in a desired direction with a reasonable amount of force.

There was difficulty in getting the TML approximation device through both ends of the tendon from the distal side. The angle of the cannula when inserted to the site forms a shallow angle with respect to the surface of the body. Due to this undesired angle, the clinician had to re-enter the tendon multiple times to try and find a route to the opposing end. This was difficult to achieve and causes additional tissue damage. The cannula ideally should become parallel with the surface considering the tendon is also parallel to the surface.

When deployed, the TML approximation device was able to successfully connect to the desired tissue which could then be pulled with reasonable force. Upon retraction of the tines, the TML approximation device was easily removed from the tissue. Overall the general design and concept of the TML approximation device for attaching to the tissue was successful.

To overcome the insertion angle obstacle, a curve may be implemented in the cannula piece that would allow the clinician to direct the TML approximation device horizontal to the surface of the body. This should better align the TML approximation device with the natural anatomy of the tendon.

Various embodiments of the disclosure could also include permutations of the various elements recited in the claims as if each dependent claim was a multiple dependent claim incorporating the limitations of each of the preceding dependent claims as well as the independent claims. Such permutations are expressly within the scope of this disclosure.

While the disclosed embodiments have been particularly shown and described with reference to a number of embodiments, it would be understood by those skilled in the art that changes in the form and details may be made to the various embodiments disclosed herein without departing from the scope of the disclosed embodiments and that the various embodiments disclosed herein are not intended to act as limitations on the scope of the claims.

The description of the various embodiments has been presented for purposes of illustration and description, but is not intended to be exhaustive or limiting of the embodiments to the form disclosed. The scope of the present disclosure is limited only by the scope of the following claims. Many modifications and variations will be apparent to those of ordinary skill in the art. The embodiments described and shown in the figures was chosen and described in order to best explain the principles of the disclosed embodiments, the practical application, and to enable others of ordinary skill in the art to understand the various embodiments with various modifications as are suited to the particular use contemplated.

## Claims

1. A tendon-muscle-ligament approximation system comprising:
a hollow tube (101) having a distal portion;
an inner core (103), housed within the hollow tube, the inner core being movable within the hollow tube between a first position and a second position, wherein the inner core comprises a distal tip (105) configured to secure biological tissue and wherein placing the inner core (103) in the first position causes the distal tip (105) to be deployed outside of the distal portion of the hollow tube (101), and wherein placing the inner core (103) in the second position causes the distal tip (105) to be retracted into the interior of the hollow tube (101);
a device control (211) in mechanical communication with the hollow tube (101) and the inner core (103) providing for the inner core (103) to be selectively placed in the first position and the second position;
**characterized by**
a stabilizer (215) operatively associated with the hollow tube (101) positioned to abut a patient skin surface when the hollow tube (101) is inserted to a selected depth into biological tissue; wherein the stabilizer (215) is selectively lockable into a position along an exterior surface of the hollow tube (101).

2. The tissue approximation system of claim 1 wherein the distal portion of the hollow tube (101) is closed.

3. The tissue approximation system of claim 2 wherein the distal portion of the hollow tube (101) comprises at least one of a Quincke, Sprotte, Touhy, beveled, Whitacre, and pencil point tip type.

4. The tissue approximation system of claim 1 wherein the distal portion of the hollow tube (101) comprises a distal opening at the distal end of the hollow tube.

5. The tissue approximation system of claim 1 wherein the hollow tube (101) defines more than a single lumen.

6. The tissue approximation system of claim 5 wherein the hollow tube (101) comprises a first lumen housing the inner core (103) and a second lumen providing for the injection of a substance into tissue from the hollow tube.

7. The tissue approximation system of claim 1 wherein the hollow tube (101) comprises at least one through port in at the distal end.

8. The tissue approximation system of claim 1, wherein the distal tip of the inner core (103) comprises at least one curved tine when deployed from the hollow tube.

9. The tissue approximation system of claim 1, wherein the distal tip has a deployed configuration of at least one of a single hook, a double hook, a triple hook, a rake design, a fluke, a claw, a grappling hook, a cork screw, a closed loop, an open loop, and a pincher.

10. The tissue approximation system of claim 1, wherein the distal tip of the inner core (103) comprises at least one distal tine and at least one proximal tine, wherein the distal tine and the proximal tine provide for attachment to tissue in separated locations.

11. The tissue approximation system of claim 1 wherein the hollow tube (101) comprises one or more tines formed in a wall of the hollow tube at the distal portion..

12. The tissue approximation system of claim 1 further comprising
a frame (800) configured to support and position a body part being treated.

13. The system of claim 12, further comprising:
a delivery device (851) configured to deliver a substance to a treatment site; and
an imaging device (823) configured to aid in the visualization of the distal end of the inner core.

14. The system of claim 13, wherein the delivery device (851) delivers a substance through a lumen within the hollow tube (101).

15. The system of claim 12, wherein the stabilizer (215) is connected to the frame (800).

## Patentansprüche

1. Sehnen-Muskel-Bänder-Annäherungssystem, umfassend:
ein Hohlrohr (101), das einen distalen Abschnitt aufweist;
einen inneren Kern (103), der in dem Hohlrohr aufgenommen ist, wobei der innere Kern innerhalb des Hohlrohrs zwischen einer ersten Position und einer zweiten Position bewegbar ist, wobei der innere Kern eine distale Spitze (105) umfasst, die zum Fixieren von biologischem Gewebe konfiguriert ist, und wobei ein Positionieren des inneren Kerns (103) in der ersten Position bewirkt, dass die distale Spitze (105) außerhalb des distalen Abschnitts des Hohlrohrs (101) verwendet wird,
und wobei ein Positionieren des inneren Kerns (103) in der zweiten Position bewirkt, dass die distale Spitze (105) in das Innere des Hohlrohrs (101) hinein zurückgezogen wird;
eine Vorrichtungssteuerung (211), die mit dem Hohlrohr (101) und dem inneren Kern (103) in mechanischer Verbindung steht, und ein selektives Positionieren des inneren Kerns (103) in der ersten und der zweiten Position bereitstellt;
**gekennzeichnet durch**
einen Stabilisator (215), der dem Hohlrohr (101) wirk-zugeordnet ist, der positioniert ist, um an der Hautoberfläche eines Patienten anzustoßen, wenn das Hohlrohr (101) in eine ausgewählte Tiefe in biologisches Gewebe eingebracht wird; wobei der Stabilisator (215) in eine Position entlang einer Außenfläche des Hohlrohrs (101) selektiv arretierbar ist.

2. Gewebeannäherungssystem nach Anspruch 1, wobei der distale Abschnitt des Hohlrohrs (101) verschlossen ist.

3. Gewebeannäherungssystem nach Anspruch 2, wobei der distale Abschnitt des Hohlrohrs (101) mindestens einen von einem Quincke-, Sprotte-, Touhy-, abgeschrägten, Whitacre- und Bleistiftspitzen-Spitzentyp umfasst.

4. Gewebeannäherungssystem nach Anspruch 1, wobei der distale Abschnitt des Hohlrohrs (101) eine distale Öffnung am distalen Ende des Hohlrohrs umfasst.

5. Gewebeannäherungssystem nach Anspruch 1, wobei das Hohlrohr (101) mehr als ein einzelnes Lumen definiert.

6. Gewebeannäherungssystem nach Anspruch 5, wobei das Hohlrohr (101) ein erstes Lumen, das den inneren Kern (103) aufnimmt, und ein zweites Lumen, das für das Injizieren einer Substanz von dem Hohlrohr in Gewebe hinein vorgesehen ist.

7. Gewebeannäherungssystem nach Anspruch 1, wobei das Hohlrohr (101) mindestens eine Durchgangsöffnung in/an dem distalen Ende umfasst.

8. Gewebeannäherungssystem nach Anspruch 1, wobei die distale Spitze des inneren Kerns (103) mindestens eine gekrümmte Zinke aufweist, wenn sie vom Hohlrohr aus verwendet wird.

9. Gewebeannäherungssystem nach Anspruch 1, wobei die distale Spitze eine Einsetz-Konfiguration von mindestens einem von einem Einfachhaken, einem Doppelhaken, einem Dreifachhaken, einer Rechenausgestaltung, einem Widerhaken, einer Klaue, einem Enterhaken, einem Korkenzieher, einer geschlossenen Schleife, einer offenen Schleife und einer Zange aufweist.

10. Gewebeannäherungssystem nach Anspruch 1, wobei die distale Spitze des inneren Kerns (103) mindestens eine distale Zinke und mindestens eine proximale Zinke umfasst, wobei die distale Zinke und die proximale Zinke ein Anbringen an Gewebe an separaten Stellen bereitstellen.

11. Gewebeannäherungssystem nach Anspruch 1, wobei das Hohlrohr (101) eine oder mehrere Zinken umfasst, die in einer Wand des Hohlrohrs im distalen Abschnitt ausgebildet sind.

12. Gewebeannäherungssystem nach Anspruch 1, ferner umfassend
einen Rahmen (800), der konfiguriert ist, einen Körperteil bei der Behandlung zu stützen und zu positionieren.

13. System nach Anspruch 12, ferner umfassend:
eine Abgabevorrichtung (851), die zur Abgabe einer Substanz an eine Behandlungsstelle konfiguriert ist; und
eine Bildgebungsvorrichtung (823), die zur Unterstützung bei der Sichtbarmachung des distalen Endes des inneren Kerns konfiguriert ist.

14. System nach Anspruch 13, wobei die Abgabevorrichtung (851) durch ein Lumen innerhalb des Hohlrohrs (101) eine Substanz abgibt.

15. System nach Anspruch 12, wobei der Stabilisator (215) mit dem Rahmen (800) verbunden ist.

## Revendications

1. Système de rapprochement tendon-muscle-ligament comprenant :
un tube creux (101) présentant une partie distale ;
un noyau interne (103) logé dans le tube creux, le noyau interne étant mobile dans le tube creux entre une première position et une seconde position, dans lequel le noyau interne comprend un embout distal (105) configuré pour fixer un tissu biologique et dans lequel le placement du noyau interne (103) dans la première position amène l'embout distal (105) à se déployer à l'extérieur de la partie distale du tube creux (101), et dans lequel le placement du noyau interne (103) dans la seconde position amène l'embout distal (105) à rentrer à l'intérieur du tube creux (101);
une commande de dispositif (211) en communication mécanique avec le tube creux (101) et le noyau interne (103) permettant au noyau interne (103) d'être placé sélectivement dans la première position et la seconde position ;
**caractérisé par**
un stabilisateur (215) associé fonctionnellement au tube creux (101) et positionné de manière à venir buter contre une surface de la peau d'un patient lorsque le tube creux (101) est inséré à une profondeur sélectionnée dans un tissu biologique ; dans lequel le stabilisateur (215) est verrouillable sélectivement dans une position le long d'une surface extérieure du tube creux (101).

2. Système de rapprochement de tissus selon la revendication 1, dans lequel la partie distale du tube creux (101) est fermée.

3. Système de rapprochement de tissus selon la revendication 2, dans lequel la partie distale du tube creux (101) comprend au moins un type d'embout parmi une aiguille de Quincke, de Sprotte, de Tuohy, un embout biseauté, une aiguille de Whiteacre et une pointe de crayon.

4. Système de rapprochement de tissus selon la revendication 1, dans lequel la partie distale du tube creux (101) comprend une ouverture distale à l'extrémité distale du tube creux.

5. Système de rapprochement de tissus selon la revendication 1, dans lequel le tube creux (101) définit plus d'une seule lumière.

6. Système de rapprochement de tissus selon la revendication 5 dans lequel le tube creux (101) comprend une première lumière logeant le noyau interne (103) et une seconde lumière permettant l'injection d'une substance dans un tissu à partir du tube creux.

7. Système de rapprochement de tissus selon la revendication 1, dans lequel le tube creux (101) comprend au moins un orifice traversant à son extrémité distale.

8. Système de rapprochement de tissus selon la revendication 1, dans lequel l'embout distal du noyau interne (103) comprend au moins une dent incurvée lorsqu'il se déploie à partir du tube creux.

9. Système de rapprochement de tissus selon la revendication 1, dans lequel l'embout distal présente une configuration déployée d'au moins un élément parmi un crochet simple, un crochet double, un crochet triple, une conception de râteau, une patte, une griffe, un grappin, un tire-bouchon, une boucle fermée, une boucle ouverte et une pince à bec.

10. Système de rapprochement de tissus selon la revendication 1, dans lequel l'embout distal du noyau interne (103) comprend au moins une dent distale et au moins une dent proximale, dans lequel la dent distale et la dent proximale permettent leur fixation à un tissu dans des emplacements séparés.

11. Système de rapprochement de tissus selon la revendication 1, dans lequel le tube creux (101) comprend une ou plusieurs dents formées dans une paroi du tube creux au niveau de la partie distale.

12. Système de rapprochement de tissus selon la revendication 1, comprenant en outre
un cadre (800) configuré pour soutenir et positionner une partie du corps traitée.

13. Système selon la revendication 12, comprenant en outre :
un dispositif de distribution (851) configuré pour distribuer une substance à un site de traitement ; et
un dispositif d'imagerie (823) configuré pour aider à la visualisation de l'extrémité distale du noyau interne.

14. Système selon la revendication 13, dans lequel le dispositif de distribution (851) distribue une substance au travers d'une lumière dans le tube creux (101).

15. Système selon la revendication 12, dans lequel le stabilisateur (215) est relié au cadre (800).
